# FASCICULE DE BREVET EUROPEEN

(11) **EP 3 318 144 B1**
(45) Date de publication et mention de la délivrance du brevet: **21.08.2019**
(21) Numéro de dépôt: 17166558.1
(22) Date de dépôt: 13.04.2017
(51) Int. Cl.: A41D 13/11, A61F 9/02, A42B 1/24, A62B 17/04

(54) **CAGOULE DE PROTECTION DE TÊTE AVEC CADRE INTÉGRÉ**
KOPFSCHUTZHAUBE MIT INTEGRIERTEM RAHMEN
PROTECTIVE HOOD WITH INTEGRATED FRAME

(30) Priorité: 04.11.2016 BE 201605820; 04.11.2016 EP 16197317
(43) Date de publication de la demande: 09.05.2018
(73) Titulaire: BeMicron, 6041 Charleroi (BE)
(72) Inventeur: VANNESTE, Vincent, 7533 THIMOUGIES (BE); DE SMET, Siegfried, 9680 MAARKEDAL (BE)
(74) Mandataire: Calysta NV

(56) Documents cités:
- WO-A1-2016/083496
- FR-A1- 2 928 260
- FR-A1- 3 016 271

## Description

La présente invention se rapporte à une cagoule de protection de tête présentant :
- une face extérieure prévue pour être en contact avec un milieu environnant,
- une face intérieure prévue pour être en contact avec la tête d'un utilisateur,
- une section de passage de tête,
- une ouverture faciale délimitée par un contour au niveau des yeux de l'utilisateur, et
- une zone respiratoire,
ladite cagoule comprenant un cadre qui est relié le long du contour de ladite ouverture faciale, qui accueille une visière et qui présente une première lèvre se plaquant sur la tête de l'utilisateur au niveau d'une zone située entre la racine et la pointe du nez, au niveau de la région frontale, au niveau des régions temporales et au niveau des régions infra-orbitaires du visage de l'utilisateur, en formant ainsi un compartiment visuel séparé de ladite zone respiratoire.

Une telle cagoule de protection de tête est connue du document FR3016271 qui divulgue une cagoule présentant une face extérieure prévue pour être en contact avec un milieu environnant, une face intérieure prévue pour être en contact avec la tête d'un utilisateur, une section de passage de tête et une ouverture faciale délimitée par un contour au niveau des yeux de l'utilisateur. Selon ce document antérieur, la cagoule est en outre munie d'une lunette de protection à cadre monture assurant un espacement entre une visière et l'appui sur le visage de l'utilisateur, la lunette de protection étant fixée sur la cagoule au niveau de l'ouverture faciale de telle sorte que la lunette et la cagoule forment un seul article. Plus particulièrement, la monture est fixée sur le bord de l'ouverture faciale de la cagoule par le bord (lèvre) de la monture qui se plaque et prend appui contre le visage de l'utilisateur. Par exemple comme illustré dans ce document antérieur, le bord (la lèvre) destinée à venir en appui sur le visage de l'utilisateur est cousu sur le pourtour de l'ouverture faciale (ouverture de la cagoule) de sorte que la cagoule et la lunette de protection ne forment qu'un seul et même article. Par ailleurs, une partie de la cagoule forme un masque de respiration qui peut être, lui aussi, cousu/fixé sur la partie inférieure de la lunette au niveau du bord (lèvre) d'appui. Notons qu'il n'est pas prévu, selon ce document antérieur, que la visière soit amovible puisqu'une procédure particulière de lavage de la cagoule/de la combinaison comprenant une cagoule est proposée pour protéger la visière durant une étape de lavage du vêtement.

Ce type de cagoule de protection de tête est particulièrement utilisé dans des environnements aseptiques et stériles où l'utilisateur doit être isolé du milieu environnant. Ceci est particulièrement indiqué dans des environnements tels que les salles blanches et les laboratoires où se préparent vaccins et médicaments. D'une part, dans ce type d'environnement, il convient d'isoler au mieux l'utilisateur afin qu'il ne contamine pas l'environnement dans lequel il travaille et, d'autre part, il s'agit parfois d'isoler l'utilisateur lui-même de substances dangereuses et/ou nocives.

Lorsqu'un opérateur est amené à travailler dans un environnement stérile, il doit particulièrement veiller à ne pas contaminer l'équipement qu'il revêt. Ainsi, l'opérateur doit respecter des procédures d'habillage strictes, lesquelles n'ont de sens que si l'équipement de protection qu'il utilise est lui-même tout à fait stérile. A cette fin, les combinaisons et les cagoules sont généralement fournies après stérilisation, dans des emballages étanches dont une ouverture aisée permet une saisie des équipements stériles. Par ailleurs, en ce qui concerne plus particulièrement les cagoules munies d'une visière, il faut que cette dernière soit non seulement stérile mais aussi parfaitement propre pour assurer une vision correcte et un confort de travail à l'utilisateur.

Malheureusement, même si une cagoule selon le document FR 3016271 présente certains avantages dont une réduction de la présence et de la formation de buée, il s'avère qu'elle n'est pas optimale et qu'elle est sujette à une usure rapide. En effet, une première problématique rencontrée avec une cagoule selon ce document antérieur réside dans le fait que le bord (lèvre) prenant appui sur le visage de l'utilisateur comporte généralement des points de couture fixant la lunette de protection à la cagoule. Ces points de couture sont en contact direct avec la peau du visage de l'utilisateur, ce qui est particulièrement gênant et peut provoquer des démangeaisons. En outre, la présence de points de couture sur le bord d'appui favorise l'entrée d'air dans le compartiment visuel puisque chaque point de couture implique que le bord d'appui est perforé, ce qui est contraire à une élimination des problèmes de formation de buée.

Une seconde problématique, quelque soit cette fois le type de fixation du cadre monture à la cagoule (couture, collage, ...), est qu'en pratique, lors des procédures de nettoyage de la cagoule à laquelle est fixée la lunette de protection, le bord (lèvre) d'appui est rapidement endommagé et de plus en plus au fil des cycles de lavage. En effet, lors d'un nettoyage en machine et/ou lors d'un séchage, la cagoule est soumise à différentes contraintes mécaniques, notamment lors des cycles rotatifs d'essorage où elle est plaquée contre les parois du tambour de la machine. Inévitablement, en pareil cas de figure, le tissu de la cagoule fixé sur le bord d'appui (lèvre) y exerce une traction provoquant son endommagement mais aussi une désolidarisation progressive de la lunette de la cagoule. Dans le cas où la liaison lunette-cagoule est réalisée par couture, les perforations dues à la couture s'agrandissent peu à peu sous l'effet des contraintes mécaniques jusqu'au moment où le bord (lèvre) d'appui fini par être déchiré par le fil de couture. Il s'en suit que le bord d'appui ne peut certainement plus assurer l'étanchéité du compartiment visuel par rapport au compartiment respiratoire et/ou que le tissu de la cagoule se détache de la lunette de protection. Ceci est évidemment problématique lorsque, comme indiqué plus haut, le but est d'isoler l'opérateur de l'environnement et inversement.

Une troisième problématique est encore que la fabrication de cagoule où la lunette est reliée par couture ou par tout autre moyen sur le bord (lèvre) d'appui est difficile à mettre en oeuvre à grande échelle : il est en effet particulièrement compliqué d'automatiser la fabrication de telles cagoules où une couture précise doit être effectuée sur un bord (lèvre) d'appui qui, de par sa nature, doit être de faible largeur. En effet, ce bord (lèvre) d'appui est destiné à épouser les formes du visage et ne doit pas être gênant pour l'utilisateur, ceci impliquant que la largeur du bord (lèvre) d'appui soit réduite tout en assurant une étanchéité adéquate entre le compartiment visuel et le compartiment respiratoire.

L'invention a pour but de pallier les inconvénients de l'état de la technique en procurant une cagoule de protection de tête pour laquelle les étapes de nettoyage et de stérilisation sont simples, ceci en parallèle avec :
- une élimination significative voire totale des problèmes de condensation et de formation de buée sur la visière ;
- un confort d'utilisation optimal, essentiellement au niveau du bord (lèvre) d'appui en contact avec la peau du visage de l'utilisateur ;
- une non dégradation (altération) de la cagoule au cours du temps lors des cycles de lavage/séchage, en particulier une non désolidarisation de la cagoule et de la lunette de protection et un non endommagement du bord (lèvre) d'appui ; et
- une facilité de fabrication de la cagoule de protection de tête, cette fabrication pouvant être automatisée et ne nécessitant pas un travail considérable ou du moins une main d'oeuvre importante.

Pour résoudre ce problème, il est prévu suivant l'invention, une cagoule de protection de tête telle qu'indiquée au début, caractérisée en ce que ledit cadre comprend en outre une deuxième lèvre périphérique située entre ladite première lèvre et ladite visière, ladite deuxième lèvre périphérique s'étendant en direction dudit milieu environnant au départ dudit cadre.

Selon l'invention, tout moyen adéquat peut être utilisé afin de relier le cadre le long de l'ouverture faciale. Par exemple, la liaison cagoule-lunette (cagoule-cadre) de protection peut être réalisée par couture, par collage ou encore par tout autre moyen permettant d'assurer un lien entre ces deux éléments. Selon un premier exemple, la deuxième lèvre périphérique du cadre peut être cousue à la cagoule le long du contour de l'ouverture faciale. Le cadre peut aussi, selon un second exemple, être relié (fixé) de façon définitive à la cagoule par pinçage du tissu formant la cagoule entre deux parties de la deuxième lèvre périphérique, ceci le long de l'ouverture faciale. Dans ce dernier cas, la deuxième lèvre périphérique peut être, selon l'invention, formée de deux parties de lèvre qui sont aptes à s'emboiter l'une dans l'autre (par exemple via un système mâle-femelle), le tissu de la cagoule et en particulier le pourtour de l'ouverture faciale de la cagoule étant alors pincé entre ces deux parties de lèvre de la deuxième lèvre périphérique.

Une telle cagoule selon l'invention, dès lors que le cadre est munie d'une deuxième lèvre périphérique située entre ladite première lèvre et ladite visière, ladite deuxième lèvre périphérique s'étendant en direction dudit milieu environnant au départ dudit cadre, est non seulement confortable mais également durable et aisée à fabriquer notamment par automatisation de certaines étapes de fabrication.

En effet, la présence de la deuxième lèvre périphérique selon l'invention permet, lorsque la liaison cagoule-lunette de protection (cagoule-cadre) est réalisée par couture, d'éviter que des points de couture ne prennent appui contre la peau de l'utilisateur. En outre la présence de la deuxième lèvre périphérique selon l'invention permet d'éliminer les problématiques d'altération/d'endommagement du bord (lèvre) d'appui et facilite grandement la réalisation même de la cagoule selon l'invention. Plus spécifiquement, le tissu de la cagoule est relié (par exemple cousu ou collé) à la deuxième lèvre périphérique selon l'invention et non pas à la première lèvre se plaquant sur la tête de l'utilisateur : la deuxième lèvre étant spécifiquement adaptée au travail de couture et/ou de collage lors de la confection ou de la fabrication, aucune couture ne touche l'utilisateur dans la zone du visage où se plaque la première lèvre lorsque la liaison cagoule-lunette de protection est réalisée par couture ; aucune contrainte mécanique n'est exercée par le tissu formant la cagoule sur la première lèvre du cadre lors de cycles de lavage/séchage ou lors de l'habillage dès lors que la première lèvre n'est pas reliée à du tissu ; et la deuxième lèvre périphérique constitue une bordure externe du cadre où une fixation automatisée par exemple par couture ou par collage peut être réalisée, cette deuxième lèvre périphérique n'ayant pas à présenter une faible largeur comme c'est le cas pour la première lèvre d'appui.

Par ailleurs, une telle cagoule suivant l'invention est particulièrement avantageuse puisqu'elle présente non seulement une visière qui peut être placée et retirée aisément (visière amovible) du cadre mais qu'elle présente également un compartiment visuel séparé, préférentiellement de façon étanche, d'une zone respiratoire. Cette séparation de la zone respiratoire et du compartiment visuel est assurée grâce à la première lèvre du cadre qui est en contact et qui se plaque sur le visage de l'utilisateur au niveau d'une zone située entre la racine et la pointe du nez - de préférence au niveau de la voûte du nez -, au niveau de la région frontale, au niveau des régions temporales et au niveau des régions infra-orbitaires, en formant ainsi un compartiment visuel séparé d'une zone respiratoire. De préférence, ce contact et ce plaquage de la première lèvre du cadre sont réalisés sur l'ensemble du pourtour du compartiment visuel de telle sorte à véritablement isoler, de préférence à isoler de façon étanche, le compartiment visuel d'une zone respiratoire. Par ailleurs, la cagoule selon l'invention assure une vision optimale en éliminant la présence d'angles morts et en assurant une vision périmétrique sur chacun des côtés de la visière, notamment à la partie basse de cette dernière.

Par les termes « zone respiratoire », on entend, au sens de la présente invention, une zone qui comprend les narines et la bouche de l'opérateur, cette zone pouvant par exemple être constituée de tissu ou au contraire être exempte de tissu. Dans le premier cas, les narines et la bouche de l'opérateur sont inclues dans la cagoule. Dans le second cas, les narines et la bouche de l'opérateur peuvent être protégées par un masque distinct de la cagoule, par exemple par un masque jetable, ce qui n'empêche toutefois pas que se pose la problématique de buée et de condensation au niveau de la visière.

Par les termes « compartiment visuel », on entend donc, au sens de la présente invention, un compartiment délimité par les parois supérieure, inférieure et latérales du cadre mais aussi par la visière fermant le cadre devant les yeux de l'utilisateur et par une zone du visage de l'opérateur délimitée par la première lèvre du cadre se plaquant au niveau d'une zone située entre la racine et la pointe du nez - de préférence au niveau de la voûte du nez -, au niveau de la région frontale, au niveau des régions temporales et au niveau des régions infra-orbitaires. De préférence, selon l'invention, le compartiment visuel ainsi délimité est prévu de telle sorte que les utilisateurs portant des lunettes correctrices puissent utiliser la cagoule sans être gênés par une visière qui s'appuierait contre la monture des lunettes.

Avec une cagoule selon l'invention, la problématique de formation de buée et de condensation est en outre significativement réduite voire totalement éliminée puisque le compartiment visuel, formé par le cadre plaqué via la première lèvre sur le visage de l'opérateur et par la visière fermant le cadre, est totalement isolé, préférentiellement isolé de façon étanche, de la zone respiratoire et donc de l'air expiré par l'opérateur.

Une cagoule suivant l'invention permet en outre de simplifier fortement les étapes de nettoyage et de stérilisation des équipements de protection. En effet, le fait de pouvoir retirer de façon simple et rapide la visière (visière amovible) du cadre de la cagoule permet en final de disposer d'une part d'un élément formé par la cagoule munie de son cadre et, d'autre part, d'un élément en un matériau plastique (ou en tout autre matériau adéquat) constitué par la visière. La visière peut être prévue en usage unique et donc être jetée et remplacée après chaque utilisation. Par ailleurs, il est possible de diriger la cagoule et son cadre dépourvu de sa visière vers une installation de traitement classique de nettoyage et/ou de stérilisation (décontamination) alors que la visière peut être acheminée, en vue de sa réutilisation, vers un système de nettoyage adéquat qui ne l'endommagera pas. Ceci est important puisque les visières sont généralement formées en un matériau plastique transparent qui ne peut pas être soumis à une stérilisation classique et encore moins une succession de stérilisations telles que celles auxquelles doit être pourtant soumise la cagoule : la visière pourrait perdre sa transparence, par exemple sous l'effet de la chaleur ou sous l'effet de rayonnements spécifiques utilisés lors de la stérilisation. Les rayons γ provoque, à titre d'exemple, un jaunissement rapide d'une visière en matière plastique après seulement quelques cycles de stérilisation. Bien entendu, une alternative est de simplement procéder au remplacement de la visière usagée par une nouvelle visière propre sur les cagoules une fois décontaminées.

Avantageusement, selon l'invention, le cadre présente une première partie de cadre s'étendant vers le milieu environnant au départ d'une face extérieure de ladite deuxième lèvre périphérique et une deuxième partie de cadre s'étendant vers la tête de l'utilisateur au départ d'une face intérieure de ladite deuxième lèvre périphérique, ladite deuxième partie de cadre étant munie de ladite première lèvre.

De préférence, selon l'invention, ladite première partie de cadre présente des orifices de passage d'air. De tels orifices de passage d'air, préférentiellement présents au niveau des parois supérieures et/ou inférieures du cadre et en retrait par rapport à la visière, permettent d'éliminer plus encore mais sans toutefois être indispensables la problématique de buée et de condensation s'accumulant au niveau de la visière. En effet, il est possible que l'utilisateur transpire quelque peu à l'intérieur du compartiment visuel, ce qui pourrait conduire également à une présence de buée et de condensation sur la visière. Toutefois, grâce aux orifices de passage d'air présents sur le cadre selon l'invention, un flux d'air est assuré dans le compartiment visuel de telle façon à éviter plus encore toute formation de buée et de condensation. Ce flux d'air est naturel puisque l'air chaud qui est contenu dans le compartiment visuel a tendance à s'évacuer via les orifices présents sur la paroi supérieure du cadre, ceci entrainant une aspiration naturelle d'air frais au départ du milieu environnant (de la salle blanche) et via les orifices présents sur la paroi inférieure du cadre : il en résulte que la température de l'air dans le compartiment visuel et dans le milieu environnant sont quasiment identiques, ce qui minimise voire élimine la présence de buée et de condensation.

Avantageusement, selon l'invention, ladite première lèvre est une lèvre continue et/ou souple. Le fait que la première lèvre du cadre soit continue et/ou souple permet d'assurer une meilleure étanchéité voire une étanchéité totale du compartiment visuel sur l'ensemble de son pourtour, ce qui permet de renforcer plus encore son isolement par rapport à la zone respiratoire.

De préférence, selon l'invention, ledit cadre fait partie intégrante de la cagoule. Comme indiqué plus haut, le cadre peut être relié le long du contour de l'ouverture faciale de la cagoule de façon définitive, ceci par exemple par l'intermédiaire d'une couture ou par collage ou par tout autre moyen adéquat. Notons que, selon l'invention, seule la visière doit être retirée avant de soumettre la cagoule à des étapes de nettoyage et de stérilisation non compatibles avec la visière. Il ne convient donc pas de replacer chaque fois un cadre sur une cagoule stérilisée mais simplement de fixer de façon amovible une visière au niveau du cadre, ce qui est rapide et permet de minimiser les manipulations à postériori, ceci par exemple lors des procédures d'habillage.

Préférentiellement, selon l'invention, ledit cadre est formé en un matériau résistant à des moyens de stérilisation tels que les rayons gamma ou β, les passages à l'autoclave ou les traitements à l'oxyde d'éthylène. Par exemple, le cadre selon l'invention peut être formé en silicone ou en polyuréthane thermoplastique (TPU) même si tout autre type de matériau adéquat tombe sous le couvert de la présente invention.

Avantageusement, la cagoule de protection de tête selon l'invention comprend un système d'auto-ajustement dudit cadre au niveau du visage de l'opérateur, ledit système d'auto-ajustement étant placé à l'arrière ou sur au moins un des côtés de la cagoule. Un tel système d'auto-ajustement du cadre permet de positionner le cadre de telle façon qu'il épouse parfaitement le visage de l'opérateur, typiquement au niveau d'une zone située entre la racine et la pointe du nez (de préférence au niveau de la voûte du nez), au niveau de la région frontale, au niveau des régions temporales et au niveau des régions infra-orbitaires. A cette fin, au moins une sangle reliée au tissu sur le côté de la cagoule ou reliée au cadre lui-même est présente pour que l'opérateur puisse positionner correctement la cadre par rapport à sa zone visuelle. En outre, la présence d'un tel système d'auto-ajustement permet d'exercer une pression de serrage uniformément répartie, ce qui assure un confort pour l'utilisateur mais aussi une étanchéité ou du moins une étanchéité quasiment totale du compartiment visuel.

Préférentiellement, selon l'invention, ladite visière comprend un système d'attache au cadre. Selon l'invention, la visière peut être placée et retirée soit par l'intérieur ou par l'extérieur de la cagoule voire indifféremment par l'intérieur ou l'extérieur. Afin que la visière soit maintenue correctement au niveau du cadre selon l'invention, elle peut être glissée dans une gorge présente au niveau de cadre ou s'emboiter selon un système mâle-femelle, les parties mâle et femelle se trouvant l'une ou l'autre indifféremment soit au niveau du cadre, soit au niveau de la visière. Dans ce dernier cas, la visière peut par exemple être munie d'ergots se glissant dans les encoches présentes sur le pourtour du cadre.

De préférence, suivant l'invention, ladite visière est en un matériau plastique transparent ou en tout autre matériau adéquat, par exemple en polycarbonate. Ce matériau peut être par exemple rigide ou flexible, teinté ou non, anti-griffe ou non, résistant aux acides ou non, selon les domaines d'application. Il est bien entendu que la visière suivant l'invention peut présenter bien d'autres propriétés.

Avantageusement, selon l'invention, le cadre est muni d'un système de respiration active, c'est-à-dire d'un système qui prélève l'ai extérieur afin de l'injecter/ l'introduire dans la zone respiratoire (compartiment respiratoire).

De préférence, la cagoule de protection de tête selon l'invention fait partie intégrante d'un vêtement de protection pour salle blanche et analogue. En effet, la cagoule suivant l'invention peut tout aussi bien former une pièce unique ou faire partie intégrante d'un vêtement de protection comme par exemple d'une combinaison de protection intégrale. Il est bien entendu que la cagoule peut être reliée par tout moyen de fixation adéquat au vêtement de protection, par exemple en y étant cousue ou en y étant fixée à l'aide d'une tirette, de boutons pressions ou encore à l'aide de tout autre moyen de fixation adéquat.

Avantageusement, la cagoule de protection de tête selon l'invention fait partie intégrante d'un vêtement de protection étant une combinaison telle que décrite dans le document EP2303044 comprenant une face extérieure prévue pour être en contact avec un milieu environnant et une face intérieure agencée pour être en contact avec un manipulateur, une première et une deuxième section de jambe reliées l'une à l'autre par une ouverture dans une zone d'entrejambe, une première et une deuxième manche et une partie de corps à laquelle sont reliées les première et deuxième manches par une extrémité liée et les première et deuxième section de jambe également par une extrémité liée, ladite partie de corps comprenant une section de passage de tête, ladite combinaison comprenant en outre au moins une zone de préhension en contact avec la face intérieure dans la partie de corps.

De préférence, la cagoule de protection de tête selon l'invention fait partie intégrante d'un vêtement de protection étant une combinaison dont l'ouverture est située dans la partie de corps, par exemple au niveau du dos.

D'autres formes de réalisation d'une cagoule de protection de tête suivant l'invention sont indiquées dans les revendications annexées.

D'autres caractéristiques, détails et avantages de l'invention ressortiront de la description donnée ci-après, à titre non limitatif et en faisant référence aux dessins annexés.
La figure 1A est une vue de face d'une cagoule de protection de tête selon l'état de la technique.
La figure 1B est une vue en coupe et de détail selon l'axe II d'une cagoule de protection de tête selon l'état de la technique.
Les figures 2A, 2B et 2C sont des vues de face de différents modes de réalisation de cagoules de protection de tête selon l'invention.
Les figures 3A, 3B et 3C sont des vues en coupe et de détail respectivement selon les axes IIIA, IIIB et IIIC des modes de réalisation des figures 2A, 2B et 2C.
La figure 4 est une vue en perspective illustrant un cadre pour une cagoule de protection de tête selon l'invention.
Les figures 5A et 5B illustrent une cagoule de protection de tête selon l'invention qui est reliée et fait partie intégrante d'un vêtement de protection.

Sur les figures, les éléments identiques ou analogues portent les mêmes références.

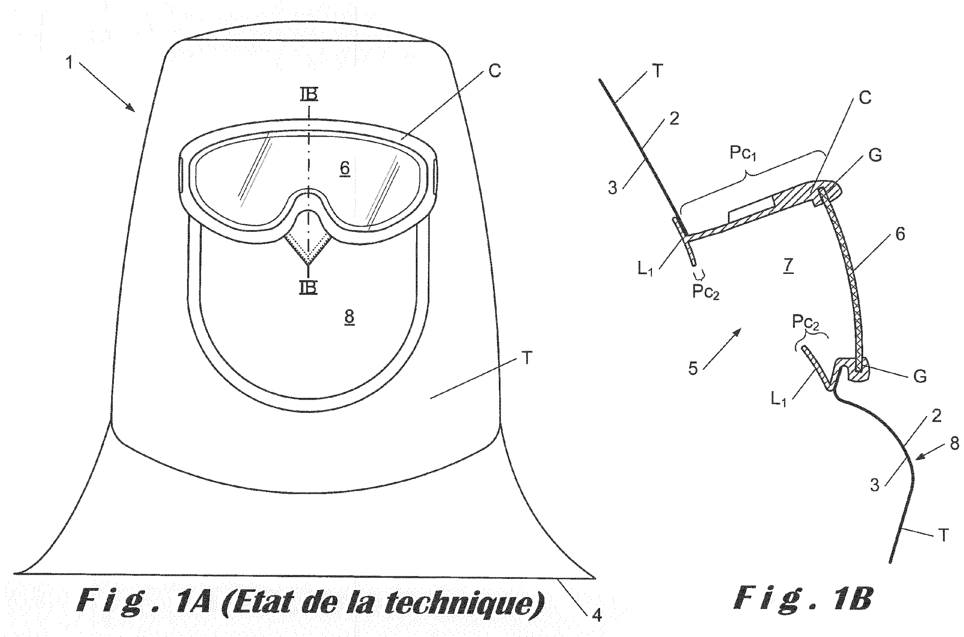

La figure 1A illustre une cagoule 1 selon l'état de la technique qui présente une face extérieure 2 prévue pour être en contact avec le milieu environnant, une face intérieure 3 prévue pour être en contact avec la tête d'un utilisateur, une section de passage de tête 4 et une ouverture faciale 5 au niveau des yeux de l'utilisateur. L'ouverture faciale 5 est délimitée par un contour le long duquel est relié un cadre C agencé pour accueillir une visière 6. Le cadre présente une lèvre L1 (non visible à la figure 1A mais visible à la figure 1B) se plaquant sur la tête de l'utilisateur au niveau d'une zone située entre la racine et la pointe du nez, au niveau de la région frontale, au niveau des régions temporales et au niveau des régions infra-orbitaires du visage de l'utilisateur, formant ainsi un compartiment visuel 7 séparé d'une zone respiratoire 8. Un tel plaquage de la lèvre L1 du cadre C permet de définir et d'isoler un compartiment visuel 7 d'une zone respiratoire 8.

Comme illustré à la figure 1B, le cadre C est relié au tissu T formant la cagoule 1 le long du contour de l'ouverture faciale 5 et présente une gorge G dans laquelle est insérée une visière 6. Le cadre C comprend en outre une lèvre L1 à laquelle est relié le tissu T de la cagoule 1, cette lèvre L1 étant continue et étant prévue, lorsque la cagoule 1 est portée par un utilisateur, pour être en contact et pour se plaquer contre le visage de l'utilisateur au niveau d'une zone située entre la racine et la pointe du nez, au niveau de la région frontale, au niveau des régions temporales et au niveau des régions infra-orbitaires, formant ainsi un compartiment visuel 7 séparé d'une zone respiratoire 8. Plus particulièrement, le cadre C présente une première partie de cadre Pc1 s'étendant vers le milieu environnant au départ de la face extérieure 2 de la cagoule 1 et une deuxième partie de cadre Pc2 s'étendant vers la tête de l'utilisateur au départ de la face intérieure 3 de la cagoule 1, la deuxième partie de cadre Pc2 étant munie de ladite lèvre L1.

Comme illustré, avec une telle cagoule 1 de protection de tête selon l'état de la technique, la face interne 3 de la cagoule 1 est reliée sur la lèvre L1. Ainsi, lorsque la liaison cagoule 1 - cadre C est réalisée par couture, des points de couture (non illustrés) sont en contact direct avec la peau de l'utilisateur et provoquent des démangeaisons. Par ailleurs, les coutures imposent que la lèvre L1 est perforée, ce qui constitue des points d'entrée d'air et ne permet pas d'assurer une étanchéité correcte du compartiment visuel 7. En outre, que la liaison cagoule 1 - cadre C soit réalisé par couture, par collage ou par tout autre moyen sur la lèvre L1, ceci implique que cette dernière est sujette à des contraintes mécaniques, le tissu T tirant sur la lèvre L1 par exemple lors de cycles de nettoyage/séchage voire même lors de la procédure d'habillage. Il en résulte que la lèvre L1 est altérée (endommagée) au cours du temps et que la cagoule 1 de protection de tête selon l'état de la technique n'est pas durable. Notons encore qu'il n'est pas aisé de relier la cagoule 1 à la lèvre L1 dès lors que la lèvre L1 se plaquant contre la tête de l'utilisateur doit être relativement fine et étroite sans quoi elle serait gênante pour l'utilisateur.

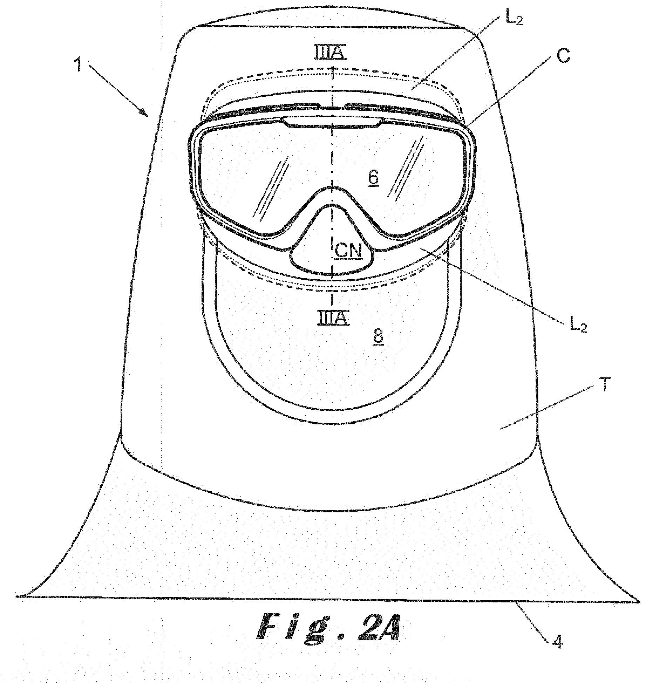

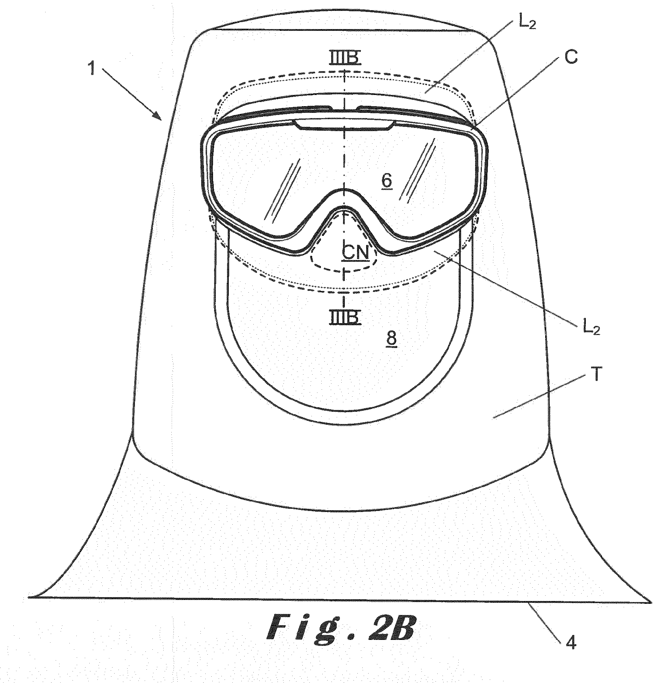

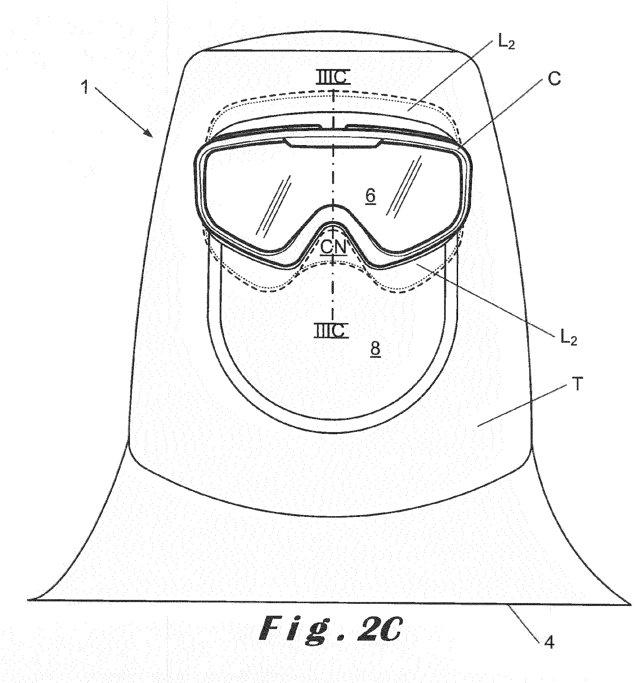

Les figures 2A, 2B et 2C illustrent trois modes de réalisation d'une cagoule de protection de tête selon l'invention. Dans le premier mode de réalisation selon la figure 2A, la cagoule 1 de protection de tête comprend un cadre C muni d'une première lèvre L1 (non visible) et d'une deuxième lèvre périphérique L2 située entre la première lèvre L1 et la visière 6, la deuxième lèvre périphérique L2 s'étendant en direction du milieu environnant. Sur cette deuxième lèvre périphérique L2, le tissu T formant la cagoule 1 est fixé par exemple par couture ou par collage. Bien entendu, le tissu T formant la cagoule 1 peut être fixé sur la face externe L2ex ou sur la face interne L2int de la lèvre périphérique L2. Comme illustré, selon ce premier mode de réalisation, le cadre C comprend un cache-nez CN qui fait partie intégrante de la lunette de protection (c'est-à-dire du cadre C). Le nez de l'opérateur est donc confiné dans le cache-nez CN qui est en un matériau identique ou non à celui du cadre C.

La figure 2B reprend les mêmes caractéristiques que celles mentionnées à la figure 2A à la différence près que le cache-nez CN selon ce deuxième mode de réalisation est constitué de tissu T, lequel est relié à la lèvre périphérique L2 par exemple par couture ou par collage.

Selon un troisième mode de réalisation illustré à la figure 2C qui reprend les mêmes éléments que ceux des deux figures précédentes, la cagoule 1 est munie d'un cache-nez CN partiel qui recouvre en partie le nez.

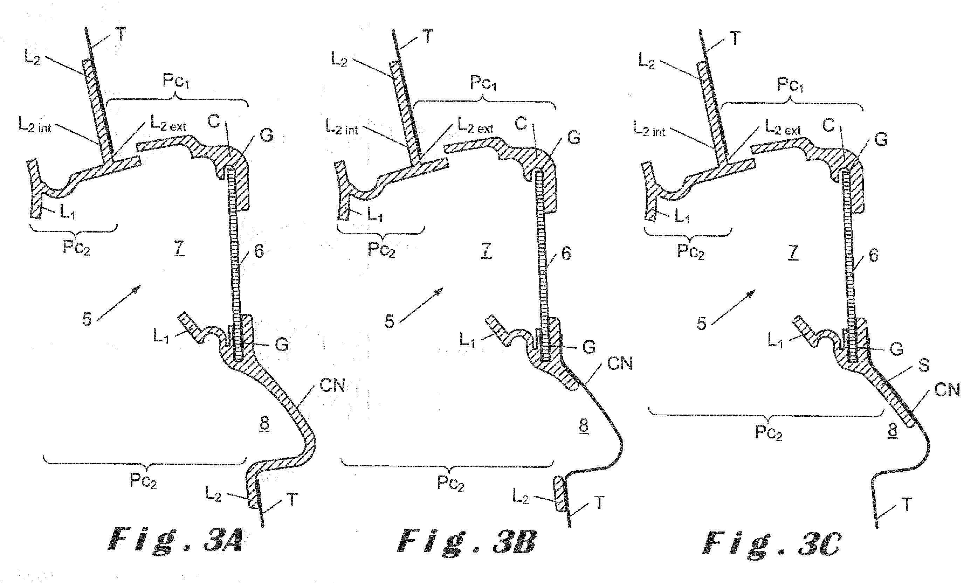

Les figures 3A, 3B et 3C sont des vues en coupe respectivement selon les axes IIIA, IIIB et IIIC des figures 2A, 2B et 2C. Comme illustré à la figure 3A, le cadre C est relié au tissu T formant la cagoule 1 le long du contour de l'ouverture faciale 5 et présente une gorge G dans laquelle est insérée une visière 6. Bien entendu, tout autre moyen de fixation de la visière 6 au cadre C fait partie intégrante de la présente invention comme par exemple une fixation par clipsage (emboîtement) ou autre. Le cadre C comprend une première lèvre L1 continue et étant prévue, lorsque la cagoule 1 est portée par un utilisateur, pour être en contact et pour se plaquer contre le visage de l'utilisateur au niveau d'une zone située entre la racine et la pointe du nez, au niveau de la région frontale, au niveau des régions temporales et au niveau des régions infra-orbitaires, formant ainsi un compartiment visuel 7 séparé d'une zone respiratoire 8. Le cadre C comprend également une deuxième lèvre périphérique L2 présentant une face externe L2ext et une face interne L2int, le tissu T de la cagoule 1 étant, selon ce mode de réalisation, fixé/relié (par exemple par couture ou par collage) à la face externe L2ext de la deuxième lèvre périphérique L2. Il est bien entendu que le tissu T de la cagoule 1 peut être, selon une alternative suivant l'invention, fixé/relié (par exemple par couture ou par collage) à la face interne L2int de la deuxième lèvre périphérique L2. Plus particulièrement, le cadre C présente une première partie de cadre Pc1 s'étendant vers le milieu environnant au départ de la face extérieure L2ext de la deuxième lèvre périphérique L2 et une deuxième partie de cadre Pc2 s'étendant vers la tête de l'utilisateur au départ de la face intérieure L2int de la deuxième lèvre périphérique L2, la deuxième partie de cadre Pc2 étant munie de ladite première lèvre L1. Selon ce premier mode de réalisation, la zone respiratoire 8 est en partie formée par une partie cache-nez CN du cadre C reliée à la deuxième lèvre périphérique L2, ce cache-nez CN englobant le nez de l'utilisateur.

La figure 3B illustre un second mode de réalisation selon l'invention et reprend les mêmes éléments que ceux illustrés à la figure 3A, à la différence près que le cache-nez CN selon ce deuxième mode de réalisation est constitué de tissu, lequel est relié d'une part à la lèvre périphérique L2 et, d'autre part à une partie de cadre proche de la visière 6, par exemple par couture ou par collage. Selon une alternative du mode de réalisation illustré à la figure 3B suivant l'invention, un cache-nez CN peut ne pas être présent (pas de tissu devant le nez de l'utilisateur) et l'utilisateur place alors un masque (par exemple un masque jetable) devant son nez.

La figure 3C illustre un troisième mode de réalisation selon l'invention et reprend les mêmes éléments que ceux illustrés aux figures 3A et 3B, à la différence près que le cache-nez CN selon ce troisième mode de réalisation est constitué de tissu T, lequel est relié d'une part à la lèvre périphérique L2 et, d'autre part à une partie de cadre proche de la visière 6 et sous forme d'une saillie S permettant de « guider » du tissu T formant le cache-nez CN.

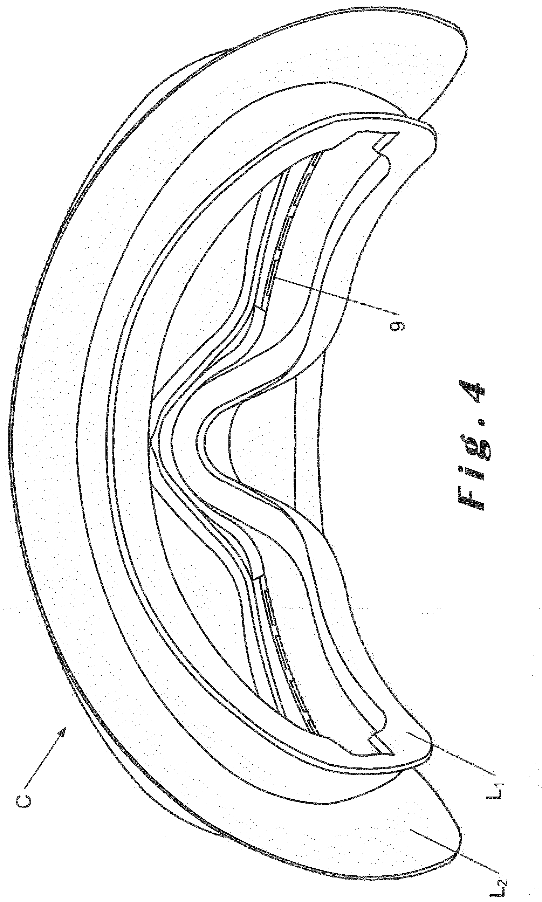

La figure 4 illustre un cadre C pour une cagoule 1 de protection de tête selon l'invention. Sur cette figure est visible la première lèvre L1 qui est continue et qui est prévue, lorsque que la cagoule 1 est portée par un utilisateur, pour être en contact et pour se plaquer contre le visage de l'utilisateur au niveau d'une zone située entre la racine et la pointe du nez, au niveau de la région frontale, au niveau des régions temporales et au niveau des régions infra-orbitaires, formant ainsi un compartiment visuel 7. Sont illustrés, au niveau de première partie de cadre Pc1 s'étendant vers le milieu environnant au départ de la face extérieure L2ext de la deuxième lèvre périphérique L2, des orifices 9 de passage d'air situés sur la paroi inférieure du cadre C. De préférence, le cadre C comporte également sur sa paroi supérieure et sur la première partie de cadre Pc1 s'étendant vers le milieu environnant au départ de la face extérieure L2ext de la deuxième lèvre périphérique L2, d'autres orifices de passage d'air (non visibles). Bien entendu, il est tout a fait envisageable, selon l'invention, que le cadre C ne soit muni d'aucun orifice de passage d'air ou que ces derniers ne soient présents qu'au niveau de la paroi supérieure ou de la paroi inférieure du cadre C.

Grâce aux orifices 9 de passage d'air présents sur le cadre C selon l'invention, un flux d'air est assuré dans le compartiment visuel 7 de telle façon à éviter plus encore toute formation de buée et de condensation. Ce flux d'air est naturel puisque l'air chaud qui est contenu dans le compartiment visuel 7 a tendance à s'évacuer via les orifices 9 présents sur la paroi supérieure du cadre C, ceci entrainant une aspiration naturel d'air frais au départ de l'environnement extérieur (par exemple de la salle blanche) et via les orifices 9 présents sur la paroi inférieure du cadre C : il en résulte que les températures de l'air dans le compartiment visuel 7 et de l'air dans l'environnement extérieur sont sensiblement identiques, ce qui contribue sans être indispensable toutefois, à éliminer la problématique de buée et de condensation au niveau de la visière 6.

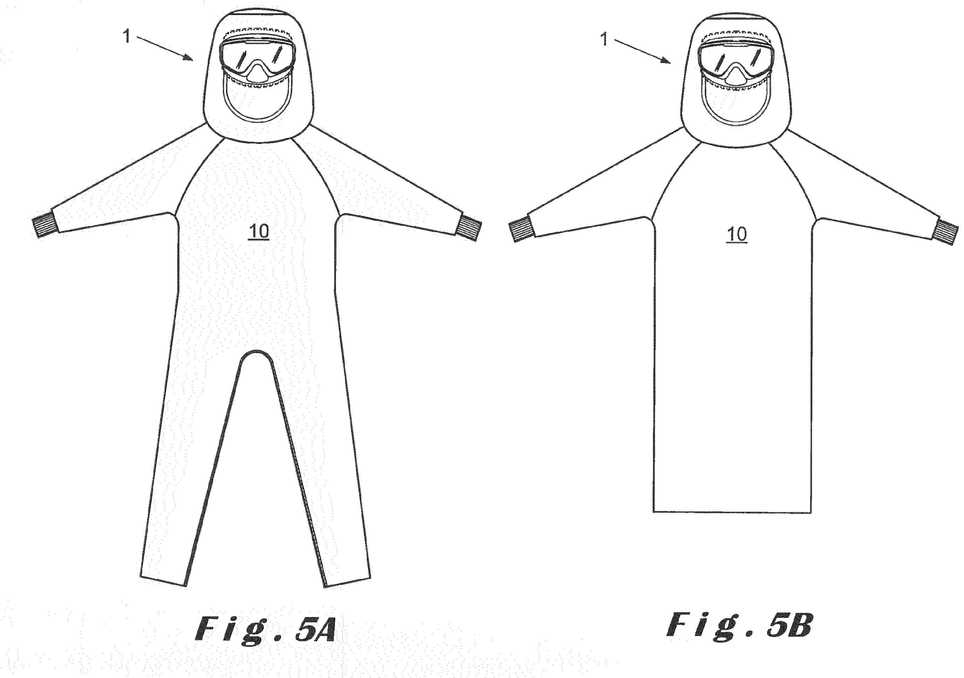

Les figures 5A et 5B illustrent une cagoule 1 selon l'invention qui est reliée et fait partie intégrante d'un vêtement de protection 10, en l'occurrence, pour l'exemple, d'une combinaison pour salles blanches et analogues. Il est bien entendu que la cagoule 1 peut être reliée par tout moyen de fixation adéquat au vêtement de protection 10, par exemple en y étant cousue ou en y étant fixée à l'aide d'une tirette ou de boutons à pression. Selon l'invention, il est également prévu que la section de passage de tête 4 soit couverte au moins partiellement par le vêtement de protection 10 de telle sorte à être insérée au moins en partie dans le vêtement de protection 10.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Cagoule (1) de protection de tête présentant :
- une face extérieure (2) prévue pour être en contact avec un milieu environnant,
- une face intérieure (3) prévue pour être en contact avec la tête d'un utilisateur,
- une section de passage de tête (4),
- une ouverture faciale (5) délimitée par un contour au niveau des yeux de l'utilisateur, et
- une zone respiratoire (8),
ladite cagoule comprenant un cadre (C) qui est relié le long du contour de ladite ouverture faciale (5), qui accueille une visière (6) et qui présente une première lèvre (L1) se plaquant sur la tête de l'utilisateur au niveau d'une zone située entre la racine et la pointe du nez, au niveau de la région frontale, au niveau des régions temporales et au niveau des régions infra-orbitaires du visage de l'utilisateur, en formant ainsi un compartiment visuel (7) séparé de ladite zone respiratoire (8), ladite cagoule étant **caractérisée en ce que** ledit cadre (C) comprend en outre une deuxième lèvre périphérique (L2) située entre ladite première lèvre (L1) et ladite visière (6), ladite deuxième lèvre périphérique (L2) s'étendant en direction dudit milieu environnant au départ dudit cadre (C).

2. Cagoule (1) de protection de tête selon la revendication 1, **caractérisée en ce que** ledit cadre (C) présente une première partie de cadre (Pc1) s'étendant vers le milieu environnant au départ d'une face extérieure (L2ex) de ladite deuxième lèvre périphérique (L2) et une deuxième partie de cadre (Pc2) s'étendant vers la tête de l'utilisateur au départ d'une face intérieure (L2int) de ladite deuxième lèvre périphérique (L2), ladite deuxième partie de cadre (Pc2) étant munie de ladite première lèvre (L1).

3. Cagoule (1) de protection de tête selon la revendication 1 ou 2, **caractérisée en ce que** ladite première partie de cadre (Pc1) présente des orifices (9) de passage d'air.

4. Cagoule (1) de protection de tête selon l'une quelconque des revendications 1 à 3, **caractérisée en ce que** ladite première lèvre (L1) est une lèvre continue et/ou souple.

5. Cagoule (1) de protection de tête selon l'une quelconque des revendications 1 à 4, **caractérisée en ce que** ledit cadre (C) fait partie intégrante de la cagoule (1).

6. Cagoule (1) de protection de tête selon l'une quelconque des revendications 1 à 5, **caractérisée en ce que** ledit cadre (C) est formé en un matériau résistant à des moyens de stérilisation tels que les rayons gamma ou β, les passages à l'autoclave ou les traitements à l'oxyde d'éthylène.

7. Cagoule (1) de protection de tête selon l'une quelconque des revendications 1 à 6, **caractérisée en ce qu'**elle comprend un système d'auto-ajustement dudit cadre (C) au niveau du visage de l'opérateur, ledit système d'auto-ajustement étant placé à l'arrière ou sur au moins un des côtés de la cagoule (1).

8. Cagoule (1) de protection de tête selon l'une quelconque des revendications 1 à 7, **caractérisée en ce que** ladite visière (6) comprend un système d'attache audit cadre (C).

9. Cagoule (1) de protection de tête selon l'une quelconque des revendications 1 à 8, **caractérisée en ce que** ladite visière (6) est en un matériau plastique transparent ou en tout autre matériau adéquat, par exemple en polycarbonate.

10. Cagoule (1) de protection de tête selon l'une quelconque des revendications 1 à 9, **caractérisée en ce qu'**elle fait partie intégrante d'un vêtement de protection (10) pour salle blanche et analogue.

11. Cagoule (1) de protection de tête selon la revendication 10, **caractérisée en ce qu'**elle fait partie intégrante d'un vêtement de protection (10) étant une combinaison comprenant une face extérieure prévue pour être en contact avec un milieu environnant et une face intérieure agencée pour être en contact avec un manipulateur, une première et une deuxième section de jambe reliées l'une à l'autre par une ouverture dans une zone d'entrejambe, une première et une deuxième manche et une partie de corps à laquelle sont reliées les premières et deuxièmes manches par une extrémité liée et les première et deuxième section de jambe également par une extrémité liée, ladite partie de corps comprenant une section de passage de tête, ladite combinaison comprenant en outre au moins une zone de préhension en contact avec la face intérieure dans la partie de corps.

12. Cagoule (1) de protection de tête selon la revendication 10, **caractérisée en ce qu'**elle fait partie intégrante d'un vêtement de protection (10) étant une combinaison dont l'ouverture est située dans la partie de corps.

## Patentansprüche

1. Kopfschutzhaube (1), die Folgendes aufweist:
- eine Außenseite (2), die dazu vorgesehen ist, sich mit einem umgebenden Medium in Kontakt zu befinden,
- eine Innenseite (3), die dazu vorgesehen ist, sich mit dem Kopf eines Benutzers in Kontakt zu befinden,
- einen Kopfdurchlassbereich (4),
- eine Gesichtsöffnung (5), die durch eine Kontur im Bereich der Augen des Benutzers begrenzt ist, und
- einen Atembereich (8),
wobei die Haube einen Rahmen (C) umfasst, der entlang der Kontur der Gesichtsöffnung (5) verbunden ist, die ein Visier (6) aufnimmt und die eine erste Lippe (L1) aufweist, die sich in einem zwischen der Nasenwurzel und der Nasenspitze befindlichen Bereich, im Frontalbereich, in den Schläfenbereichen und in den infraorbitalen Bereichen des Gesichts des Benutzers an den Kopf des Benutzers presst, wodurch eine vom Atembereich (8) getrennte Sichtkammer (7) gebildet wird,
wobei die Haube **dadurch gekennzeichnet ist, dass** der Rahmen (C) weiter eine zweite umlaufende Lippe (L2) umfasst, die sich zwischen der ersten Lippe (L1) und dem Visier (6) befindet, wobei die zweite umlaufende Lippe (L2) sich ausgehend vom Rahmen (C) in Richtung des umgebenden Mediums erstreckt.

2. Kopfschutzhaube (1) nach Anspruch 1, **dadurch gekennzeichnet, dass** der Rahmen (C) einen ersten Rahmenteil (Pc1), der sich ausgehend von einer Außenseite (L2ex) der zweiten umlaufenden Lippe (L2) in Richtung des umgebenden Mediums erstreckt, und einen zweiten Rahmenteil (Pc2), der sich ausgehend von einer Innenseite (L2int) der zweiten umlaufenden Lippe (L2) in Richtung des Kopfes des Benutzers erstreckt, aufweist, wobei der zweite Rahmenteil (Pc2) mit der ersten Lippe (L1) versehen ist.

3. Kopfschutzhaube (1) nach Anspruch 1 oder 2, **dadurch gekennzeichnet, dass** der erste Rahmenteil (Pc1) Luftdurchtrittsöffnungen (9) aufweist.

4. Kopfschutzhaube (1) nach einem der Ansprüche 1 bis 3, **dadurch gekennzeichnet, dass** die erste Lippe (L1) eine umlaufende und/oder biegsame Lippe ist.

5. Kopfschutzhaube (1) nach einem der Ansprüche 1 bis 4, **dadurch gekennzeichnet, dass** der Rahmen (C) integraler Bestandteil der Haube (1) ist.

6. Kopfschutzhaube (1) nach einem der Ansprüche 1 bis 5, **dadurch gekennzeichnet, dass** der Rahmen (C) aus einem Material gebildet ist, das gegen Sterilisationsmittel wie Gamma- oder β-Strahlen, Autoklavierungen oder Behandlungen mit Ethylenoxid beständig ist.

7. Kopfschutzhaube (1) nach einem der Ansprüche 1 bis 6, **dadurch gekennzeichnet, dass** sie ein System zur selbständigen Anpassung des Rahmens (C) im Bereich des Gesichts des Benutzers umfasst, wobei das System zur selbständigen Anpassung an der Rückseite oder auf mindestens einer der Seiten der Haube (1) angeordnet ist.

8. Kopfschutzhaube (1) nach einem der Ansprüche 1 bis 7, **dadurch gekennzeichnet, dass** das Visier (6) ein System zur Befestigung des Rahmens (C) umfasst.

9. Kopfschutzhaube (1) nach einem der Ansprüche 1 bis 8, **dadurch gekennzeichnet, dass** das Visier (6) aus einem transparenten Kunststoffmaterial oder jedem anderen geeigneten Material, beispielsweise Polycarbonat, besteht.

10. Kopfschutzhaube (1) nach einem der Ansprüche 1 bis 9, **dadurch gekennzeichnet, dass** sie integraler Bestandteil einer Reinraum-Schutzkleidung (10) und dergleichen ist.

11. Kopfschutzhaube (1) nach Anspruch 10, **dadurch gekennzeichnet, dass** sie integraler Bestandteil einer Schutzkleidung (10) ist, bei der es sich um einen Overall handelt, der eine Außenseite, die dazu vorgesehen ist, sich mit einem umgebenden Medium in Kontakt zu befinden, und eine Innenseite, die so angeordnet ist, dass sie sich mit einem Manipulierer in Kontakt befindet, einen ersten und einen zweiten Beinabschnitt, die über eine Öffnung im Schrittbereich miteinander verbunden sind, einen ersten und einen zweiten Ärmel und einen Rumpfteil, mit dem der erste und der zweite Ärmel über ein verbundenes Ende und der erste und der zweite Beinabschnitt ebenfalls über ein verbundenes Ende verbunden sind, umfasst, wobei der Rumpfteil einen Kopfdurchlassbereich umfasst, wobei der Overall weiter mindestens einen Griffbereich in Kontakt mit der Innenseite im Rumpfteil umfasst.

12. Kopfschutzhaube (1) nach Anspruch 10, **dadurch gekennzeichnet, dass** sie integraler Bestandteil einer Schutzkleidung (10) ist, bei der es sich um einen Overall handelt, dessen Öffnung sich im Rumpfteil befindet.

## Claims

1. Hood (1) for head protection having:
- an outer face (2) intended to be in contact with the surroundings,
- an inner face (3) intended to be in contact with a user's head,
- a section through which the head passes (4),
- a face opening (5) delimited by a contour at the level of the user's eyes, and
- a breathing zone (8),
said hood comprising a frame (C) that is connected along the contour of said face opening (5), which receives a visor (6) and which has a first lip (L1) resting on the user's head at the level of a zone located between the root and the tip of the nose, at the level of the frontal region, at the level of the temporal regions and at the level of the infra-orbital regions of the user's face, thus forming a visual compartment (7) separated from said breathing zone (8), said hood being **characterised in that** said frame (C) further comprises a second peripheral lip (L2) located between said first lip (L1) and said visor (6), said second peripheral lip (L2) extending in the direction of said surroundings at the start of said frame (C).

2. Hood (1) for head protection according to claim 1, **characterised in that** said frame (C) has a first frame part (Pc1) extending towards the surroundings at the start of an outer face (L2ex) of said second peripheral lip (L2) and a second frame part (Pc2) extending towards the user's head at the start of an inner face (L2int) of said second peripheral lip (L2), said second frame part (Pc2) being provided with said first lip (L1).

3. Hood (1) for head protection according to claim 1 or 2, **characterised in that** said first frame part (Pc1) has holes (9) for passage of air.

4. Hood (1) for head protection according to any one of claims 1 to 3, **characterised in that** said first lip (L1) is a continuous and/or flexible lip.

5. Hood (1) for head protection according to any one of claims 1 to 4, **characterised in that** said frame (C) forms an integral part of the hood (1).

6. Hood (1) for head protection according to any one of claims 1 to 5, **characterised in that** said frame (C) is formed of a material that is resistant to sterilising means such as gamma or β rays, autoclaving or treatments with ethylene oxide.

7. Hood (1) for head protection according to any one of claims 1 to 6, **characterised in that** it comprises a system for automatic adjustment of said frame (C) to the level of the operator's face, said system for automatic adjustment being placed at the back or on at least one of the sides of the hood (1).

8. Hood (1) for head protection according to any one of claims 1 to 7, **characterised in that** said visor (6) comprises a system for attachment to said frame (C).

9. Hood (1) for head protection according to any one of claims 1 to 8, **characterised in that** said visor (6) is of a transparent plastic material or of any other suitable material, for example polycarbonate.

10. Hood (1) for head protection according to any one of claims 1 to 9, **characterised in that** it forms an integral part of protective clothing (10) for clean rooms and the like.

11. Hood (1) for head protection according to claim 10, **characterised in that** it forms an integral part of protective clothing (10), being a suit comprising an outer face intended to be in contact with the surroundings and an inner face arranged to be in contact with a manipulator, a first and a second leg section connected to one another via an opening in a crotch zone, a first and a second sleeve and a body part to which the first and second sleeves are connected by a connected end and the first and second leg section also by a connected end, said body part comprising a section for passage of the head, said suit further comprising at least one grip zone in contact with the inner face in the body part.

12. Hood (1) for head protection according to claim 10, **characterised in that** it forms an integral part of protective clothing (10), being a suit, the opening of which is located in the body part.
